# FASCICULE DE BREVET EUROPEEN

(11) **EP 3 714 261 B1**
(45) Date de publication et mention de la délivrance du brevet: **18.08.2021**
(21) Numéro de dépôt: 18786774.2
(22) Date de dépôt: 15.10.2018
(51) Int. Cl.: B01D 53/30, B01D 53/52, B01D 53/78, G01N 27/416, B01D 53/00, G01N 33/18

(54) **PROCEDE DE DETERMINATION DE LA CONCENTRATION EN CHLORE LIBRE DANS UNE TOUR UTILISANT LE CHLORE COMME BASE ACTIVE**
VERFAHREN ZUR BESTIMMUNG DER FREIEN CHLORKONZENTRATION IN EINEM TURM MIT CHLOR ALS AKTIVE BASE
METHOD FOR DETERMINING THE FREE CHLORINE CONCENTRATION IN A TOWER USING THE CHLORINE AS AN ACTIVE BASE

(30) Priorité: 21.11.2017 FR 1760969
(43) Date de publication de la demande: 30.09.2020
(73) Titulaire: SUEZ Groupe, 92040 Paris la Défense CEDEX (FR)
(72) Inventeur: GRIMAUD, Claude, 78450 Villepreux (FR)
(74) Mandataire: Marks & Clerk France
(86) Numéro de dépôt international: PCT/EP2018/078078
(87) Numéro de publication internationale: WO 2019/101442

(56) Documents cités:
- EP-A2- 2 669 676
- JP-A- 2010 227 381
- US-A- 5 693 204
- US-B1- 7 189 314

## Description

L'invention se situe dans le domaine de la désodorisation de l'air vicié par injection d'un produit chloré. L'invention concerne un procédé de détermination de la concentration en chlore libre d'une solution liquide. L'invention concerne également un procédé de régulation de la concentration en chlore libre dans une tour utilisant le chlore comme base active. Enfin l'invention concerne un dispositif de détermination de la concentration en chlore libre dans une solution liquide.

Dans cette demande, l'invention sera présentée dans le cas de l'air vicié par du sulfure d'hydrogène (H2S) et ses dérivés (mercaptans) et traité par injection d'un produit chloré. Néanmoins l'invention s'applique de façon similaire à tout autre type d'air vicié pouvant être traité par injection d'un produit chloré ou pour le dosage d'une solution chlorée pour toute application l'utilisant dans son procédé.

Dans les stations d'épuration, les boues biologiques fermentent, ce qui conduit à la production d'hydrogène sulfuré, un gaz toxique, fort en odeur, corrosif et explosif. Son élimination est possible en milieu humide par oxydation à partir d'un produit chloré comme l'hypochlorite de sodium (NaClO) dans une tour de mise en contact.

Une tour de mise en contact est assez haute et comprend un garnissage. L'air vicié est introduit en bas de la tour et dirigé vers le haut de la tour généralement au moyen d'un ventilateur situé indifféremment en amont ou en aval de la tour. Une solution liquide comprenant le produit chloré de traitement et stockée dans le pied de la tour est amenée en haut de la tour par pompage et ruisselle sur le garnissage. L'air vicié en remontant dans la tour entre en contact avec le milieu aqueux à contre-courant. Le garnissage a pour rôle d'accroître la surface d'échange entre l'air vicié et le produit chloré. L'écoulement diphasique permet la dissolution du sulfure d'hydrogène dans le milieu liquide et son élimination par réaction d'oxydation chimique avec le chlore contenu dans le produit chloré de traitement.

Du fait de la réaction chimique entre le sulfure d'hydrogène de l'air vicié et du chlore du produit chloré de traitement, il résulte une consommation du chlore présent dans la solution liquide. Il faut donc introduire de la solution de traitement pour maintenir constante la quantité d'oxydant, c'est-à-dire du produit chloré, dans une gamme de concentration.

L'ajustement du produit chloré de traitement doit être suffisamment précis. Si le produit chloré de traitement est introduit dans la solution liquide en trop faible quantité, le produit chloré est en défaut et il restera du sulfure d'hydrogène en sortie de la tour. Si le produit chloré de traitement est introduit dans la solution liquide en trop grande quantité, cela occasionne un coût supplémentaire du fait de la surconsommation du produit chloré et une forte odeur chlorée peut constituer une gêne pour les riverains.

Il est donc primordial de pouvoir évaluer avec précision la quantité de produit chloré à ajouter dans la solution liquide en pied de tour. Cette évaluation se fait par le suivi de la concentration en chlore libre contenu dans la solution liquide conservée dans le pied de tour.

Il existe des solutions pour déterminer la concentration de chlore libre à forte concentration dans la réserve du pied de tour.

La première solution existante consiste en la mesure par absorption de lumière dans le domaine spectral de l'ultraviolet. Un analyseur met en œuvre une méthode spectrale en mesurant par absorption de la lumière et sans utilisation de réactif permet de mesurer des concentrations entre 100 et 3000 mg/L en chlore dans des solutions de nettoyage au chlore ou de traitement pour tour de désodorisation.

Une autre solution existante consiste en une mesure ampérométrique. Cette technique utilise une sonde ampérométrique à membrane sélective au chlore actif permettant de doser le chlore dans le domaine des faibles concentrations (mg/L).

Dans US 5,693,204, une méthode différente de détermination de la concentration en chlore libre par la mesure de la quantité d'acide hypochloreux est décrite, le pH de l'échantillon est maintenu en dessous de 8 par addition d'acide citrique.

Ces solutions présentent l'inconvénient d'un investissement relativement élevé. L'entretien et la maintenance de systèmes mettant en œuvre ces méthodes sont difficiles et chronophages. Par ailleurs, la méthode ampérométrique dose le chlore dans de faibles concentrations, et n'est donc pas directement utilisable dans le domaine de concentrations élevées de l'application relative à l'invention. Une importante dilution de l'échantillon est obligatoire (x500 ou x1000).

L'invention vise à pallier tout ou partie des problèmes cités plus haut en proposant un procédé de détermination de la concentration en chlore libre contenu dans la solution liquide conservée dans le pied de tour en se basant sur la mesure de son potentiel redox afin de déterminer la concentration en chlore libre dans la solution liquide.

A cet effet, l'invention a pour objet un procédé de détermination de la concentration en chlore libre dans une première solution liquide, comprenant les étapes suivantes :
- première étape d'introduction de la première solution liquide dans un analyseur comprenant une sonde redox,
- deuxième étape de mesure du pH de la première solution liquide,
- troisième étape d'ajout de soude jusqu'à obtention d'une seconde solution liquide de pH supérieur à 12, préférentiellement de 12,5 à 12,8, la troisième étape étant préférentiellement mise en œuvre simultanément à la deuxième étape,
- quatrième étape de détermination d'une valeur du potentiel redox de la seconde solution liquide par la sonde redox,
- cinquième étape de détermination de la concentration en chlore libre dans la première solution liquide en fonction de la valeur du potentiel redox déterminé.

Ce procédé permet de déterminer la concentration en chlore libre dans une première solution de manière simple et précise en déterminant le potentiel redox de cette solution à laquelle de la soude a été ajoutée pour être dans une certaine plage de pH.

Dans un mode de réalisation, le procédé de détermination de la concentration en chlore libre selon l'invention peut comprendre entre la troisième étape et la quatrième étape une étape de temporisation. L'étape de temporisation permet une stabilisation du pH de la seconde solution liquide avant d'en déterminer la valeur de son potentiel redox. Il en résulte une meilleure précision de la valeur ainsi déterminée.

Dans un autre mode de réalisation, le procédé de détermination de la concentration en chlore libre selon l'invention peut comprendre après la quatrième étape une étape de mémorisation de la valeur du potentiel redox, permettant de sauvegarder la valeur du potentiel redox déterminée. Cette valeur sauvegardée peut ainsi être utilisée ultérieurement.

Dans un autre mode de réalisation, la quatrième étape de détermination de la valeur du potentiel redox et l'étape de mémorisation de la valeur du potentiel redox peuvent être répétées successivement selon une fréquence prédéfinie pour obtenir des valeurs mémorisées de potentiel redox, et le procédé selon l'invention peut comprendre en outre une étape de calcul d'une valeur moyenne des valeurs mémorisées de potentiel redox. Il est ainsi possible d'établir une valeur moyenne des différents potentiels redox déterminés, afin de tenir compte des éventuelles variations possibles des mesures de potentiels dans la seconde solution liquide.

Dans un autre mode de réalisation, la quatrième étape de détermination de la valeur du potentiel redox et l'étape de mémorisation de la valeur du potentiel redox sont répétées successivement jusqu'à ce que le pH de la seconde solution liquide soit inférieur à une valeur de seuil prédéfinie. Cela permet de déterminer la valeur du potentiel redox et de la mémoriser uniquement dans la plage de pH dans laquelle la sonde redox peut déterminer un potentiel redox de façon précise.

Dans un autre mode de réalisation, le procédé de détermination de la concentration en chlore libre selon l'invention peut comprendre, une fois préalablement à la première étape, une étape d'étalonnage d'une fonction reliant une concentration en chlore libre prédéterminée à une valeur du potentiel redox. L'étalonnage permet de définir la fonction permettant d'obtenir la concentration en chlore libre de la première solution à partir d'une valeur du potentiel redox de la seconde solution.

Dans un autre mode de réalisation, le procédé de détermination de la concentration en chlore libre selon l'invention peut comprendre en outre une étape de déclenchement d'une alarme si au moins une des conditions suivantes est réalisée :
- La mesure du pH de la première solution liquide est inférieure à 7 ou supérieure à 13,
- La mesure du pH de la seconde solution liquide est inférieure à 12 ou supérieure à 13,
- La valeur du potentiel redox de la seconde solution liquide est inférieure à 0,1 V ou supérieure à 0,5 V.

Le déclenchement de l'alarme permet de stopper le procédé et/ou prévenir un utilisateur d'un dysfonctionnement dans le procédé.

L'invention concerne aussi un procédé de régulation de la concentration en chlore libre d'un volume d'une première solution liquide dans une tour utilisant du chlore comme base active, comprenant :
- une étape de prélèvement d'un échantillon du volume de la première solution liquide en pied de la tour,
- les étapes du procédé de détermination de la concentration en chlore libre dans l'échantillon selon l'une quelconque des revendications précédentes,
- après la cinquième étape de détermination de la concentration en chlore libre dans l'échantillon, une étape d'ajout d'une quantité d'un produit chloré dans le volume de la première solution liquide en fonction de la concentration en chlore libre dans l'échantillon, de sorte à maintenir la concentration en chlore libre dans le volume de la première solution liquide à une valeur prédéterminée. Ce procédé permet de réguler la concentration en chlore libre dans le réservoir en pied de tour après avoir déterminé la concentration en chlore libre dans le réservoir. Il en résulte un maintien de la concentration en chlore libre dans le réservoir en pied de tour à une valeur souhaitée.

L'invention concerne aussi un dispositif de détermination de la concentration en chlore libre d'une première solution liquide, comprenant :
- un analyseur, destiné à recevoir la première solution liquide,
- un pH-mètre destiné à mesurer le pH de la première solution liquide dans l'analyseur,
- un injecteur de soude adapté à ajouter de la soude à la première solution liquide jusqu'à obtention d'une seconde solution de pH supérieur à 12, préférentiellement entre 12,5 et 12,8,
- une sonde redox, destinée à déterminer une valeur du potentiel redox de la seconde solution liquide,
- un calculateur destiné à déterminer la concentration en chlore libre dans la première solution liquide en fonction de la valeur du potentiel redox déterminé.

Le dispositif selon l'invention permet de déterminer la concentration en chlore libre contenu dans la première solution liquide en se basant sur la mesure de son potentiel redox afin de déterminer la concentration en chlore libre dans la première solution liquide.

Dans un mode de réalisation, le dispositif selon l'invention peut comprendre un moyen de mémorisation de la valeur du potentiel redox. Il est alors possible de sauvegarder la valeur du potentiel redox déterminée. Cette valeur sauvegardée peut être utilisée ultérieurement.

Dans un autre mode de réalisation, le dispositif selon l'invention, configuré pour déterminer la valeur du potentiel redox de la seconde solution liquide et mémoriser la valeur du potentiel redox successivement et de façon répétée à une fréquence prédéfinie pour obtenir des valeurs mémorisées de potentiel redox, peut comprendre un moyen de calcul d'une valeur moyenne des valeurs mémorisées de potentiel redox. L'établissement d'une valeur moyenne des différents potentiels redox déterminés permet de tenir compte des éventuelles variations possibles des mesures de potentiels dans la seconde solution liquide, et de dresser des moyennes horaires ou journalières.

Dans un autre mode de réalisation, le dispositif selon l'invention peut comprendre une alarme configurée pour se déclencher si au moins une des conditions suivantes est réalisée :
- La mesure du pH de la première solution liquide est inférieure à 7 ou supérieure à 13,
- La mesure du pH de la seconde solution liquide est inférieure à 12 ou supérieure à 13,
- La valeur du potentiel redox de la seconde solution liquide est inférieure à 0,1 V ou supérieure à 0,5 V.

Un utilisateur peut être informé de tout dysfonctionnement ayant lieu lors d'une des étapes du procédé de détermination de la concentration en chlore libre. L'utilisateur est alors en mesure de voir s'il s'agit d'un point aberrant (erreur ponctuelle de mesure) ou décider de stopper le procédé s'il le juge nécessaire.

L'invention sera mieux comprise et d'autres avantages apparaîtront à la lecture de la description détaillée d'un mode de réalisation donné à titre d'exemple, description illustrée par le dessin joint dans lequel :
- la figure 1 représente schématiquement un dispositif de détermination de la concentration en chlore libre d'une première solution liquide selon l'invention,
- la figure 2 représente schématiquement les étapes du procédé de détermination de la concentration en chlore libre d'une première solution liquide selon l'invention,
- la figure 3 représente schématiquement les étapes du procédé de régulation de la concentration en chlore libre d'un volume d'une première solution liquide dans une tour utilisant du chlore comme base active selon l'invention,
- la figure 4 représente schématiquement une tour de désodorisation mettant en œuvre un dispositif de détermination de la concentration en chlore libre selon l'invention.

Par souci de clarté, les mêmes éléments porteront les mêmes repères dans les différentes figures.

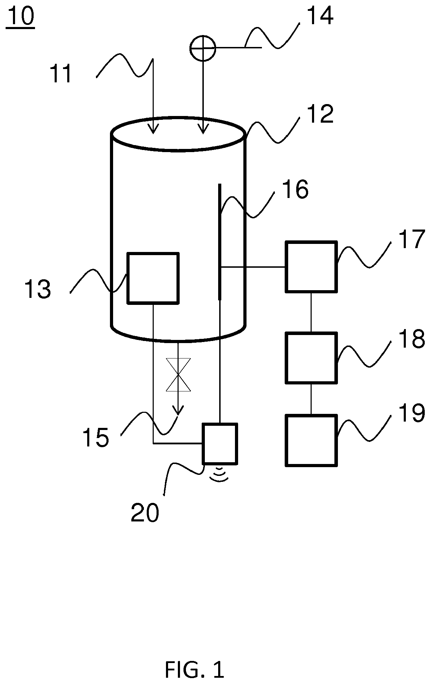

La **figure 1** représente schématiquement un dispositif 10 de détermination de la concentration en chlore libre d'une première solution liquide 11 selon l'invention. Le dispositif 10 de détermination de la concentration en chlore libre de la première solution liquide 11 comprend un analyseur 12, destiné à recevoir la première solution liquide 11 dont on souhaite déterminer la concentration en chlore libre. Le dispositif comprend un pH-mètre 13 destiné à mesurer le pH de la première solution liquide 11 dans l'analyseur 12, un injecteur de soude 14 destiné à ajouter de la soude à la première solution liquide 11 jusqu'à obtention d'une seconde solution 15 de pH supérieur à 12, préférentiellement entre 12,5 et 12,8, une sonde redox 16, destinée à déterminer une valeur du potentiel redox de la seconde solution liquide 15, et un calculateur 17 destiné à déterminer la concentration en chlore libre dans la première solution liquide 11 en fonction de la valeur du potentiel redox déterminé.

La première solution liquide 11 dont on souhaite connaitre la concentration en chlore libre est mise dans l'analyseur 12. Le pH-mètre 13 en mesure le pH. L'injecteur de soude 14 permet d'ajouter de la soude à cette première solution 11 jusqu'à obtenir la seconde solution 15 de pH supérieur à 12. Avantageusement, le pH de la seconde solution 15 est situé entre 12,5 et 12,8. L'ajout de soude est nécessaire pour avoir une solution à pH élevé puisque la sonde redox ne fonctionne pas ou mal dans des solutions à faible pH.

Une fois la seconde solution liquide 15 obtenue, avec un pH élevé, la sonde redox fournit la valeur (en millivolts) du potentiel redox de la seconde solution 15. Le calculateur 17 permet de calculer la concentration en chlore libre dans la première solution liquide 11 à partir de la valeur du potentiel redox déterminé par la sonde redox.

La sonde redox 16 consiste en une électrode spécifique pour mesurer le potentiel d'oxydoréduction de la solution liquide 15. C'est un matériel simple pour mesurer le caractère oxydoréducteur d'un milieu aqueux. Cette sonde présente l'avantage d'être peu onéreuse, suffisamment fiable et précise pour le domaine d'application concerné. Par contre, ce type de sonde n'est pas utilisable dans le domaine de fonctionnement d'une tour de désodorisation standard en raison du faible pH résultant de la concentration des produits ajoutés.

Le dispositif selon l'invention permet l'utilisation d'une telle sonde redox en modifiant le pH de la première solution par ajout de soude pour donner une seconde solution liquide de pH élevé. En fonction de la valeur du potentiel redox de cette seconde solution liquide, le calculateur 17 permet le calcul de la concentration en chlore libre dans la première solution 11.

Avantageusement, le dispositif 10 peut comprendre un moyen de mémorisation 18 de la valeur du potentiel redox. En sauvegardant la valeur du potentiel redox, le moyen de mémorisation 18 permet l'utilisation ultérieure de cette valeur, soit pour la contrôler, soit pour l'inclure dans un calcul de moyenne.

Avantageusement, le dispositif 10 est configuré pour déterminer la valeur du potentiel redox de la seconde solution liquide 15 et mémoriser la valeur du potentiel redox successivement et de façon répétée à une fréquence prédéfinie pour obtenir des valeurs mémorisées de potentiel redox. Dans ce cas, il comprend un moyen de calcul 19 d'une valeur moyenne des valeurs mémorisées de potentiel redox. Plusieurs mesures du potentiel redox de la seconde solution 15 sont réalisées et les valeurs sont sauvegardées. Ensuite, le moyen de calcul 19 permet le calcul de la moyenne d'une partie ou de la totalité des valeurs mémorisées.

Avantageusement, le dispositif 10 selon l'invention peut comprendre une alarme 20 configurée pour se déclencher si au moins une des conditions suivantes est réalisée :
- La mesure du pH de la première solution liquide est inférieure à 7 ou supérieure à 13,
- La mesure du pH de la seconde solution liquide est inférieure à 12 ou supérieure à 13,
- La valeur du potentiel redox de la seconde solution liquide est inférieure à 0,1 V ou supérieure à 0,5 V.

L'alarme permet de prévenir un utilisateur d'une situation inhabituelle, c'est-à-dire si le pH de la première solution issu de la tour de désodorisation est anormalement faible ou anormalement élevé, ou bien si le pH de la seconde solution, normalement supérieur à 12 et préférentiellement entre 12,5 et 12,8, ne se trouve pas dans la plage de pH escomptée ou enfin si la valeur du potentiel redox de la seconde solution est trop faible ou trop élevé par rapport aux valeurs attendues. Le déclenchement de l'alarme peut prévoir l'arrêt du dispositif. En complément, ou en alternative, le choix de stopper les mesures peut incomber à l'utilisateur s'il estime qu'il y a un réel dysfonctionnement du dispositif.

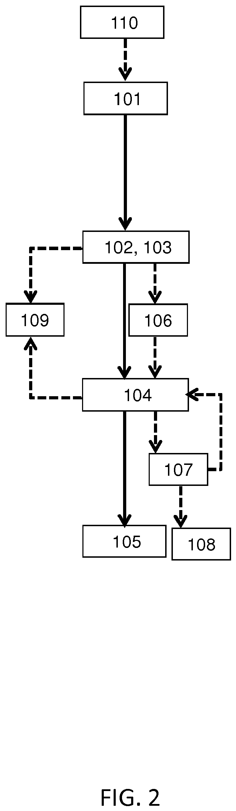

La **figure 2** représente schématiquement les étapes du procédé de détermination de la concentration en chlore libre d'une première solution liquide 11 selon l'invention. Le procédé de détermination de la concentration en chlore libre dans la première solution liquide 11 comprend les étapes suivantes :
- première étape 101 d'introduction de la première solution liquide 11 dans un analyseur 12 comprenant une sonde redox 16,
- deuxième étape 102 de mesure du pH de la première solution liquide 11,
- troisième étape 103 d'ajout de soude jusqu'à obtention d'une seconde solution liquide 15 de pH supérieur à 12, préférentiellement de 12,5 à 12,8, la troisième étape 103 étant préférentiellement mise en œuvre simultanément à la deuxième étape 102,
- quatrième étape 104 de détermination d'une valeur du potentiel redox de la seconde solution liquide 15 par la sonde redox 16,
- cinquième étape 105 de détermination de la concentration en chlore libre dans la première solution liquide 11 en fonction de la valeur du potentiel redox déterminé.

L'ajout de soude provoque une élévation rapide du pH de la solution. Dès que la valeur de pH de la solution dépasse le seuil renseigné (par exemple pH supérieur à 12,5), l'ajout de soude est interrompu.

L'avantage de la mise en œuvre d'une sonde redox, outre le fait qu'il s'agisse d'un matériel peu onéreux, réside également dans le fait que la mesure se réalise dans un milieu toujours propre, il n'y a donc aucun risque d'encrassement du matériel de mesure.

Le procédé de détermination de la concentration en chlore libre selon l'invention peut comprendre entre la troisième étape 103 et la quatrième étape 104 une étape 106 de temporisation, pendant laquelle le pH de la seconde solution 15 a le temps de se stabiliser, juste avant d'effectuer la mesure du potentiel redox de la seconde solution liquide 15.

Le procédé de détermination de la concentration en chlore libre selon l'invention peut comprendre après la quatrième étape 104 une étape 107 de mémorisation de la valeur du potentiel redox.

Avantageusement, la quatrième étape 104 de détermination de la valeur du potentiel redox et l'étape 107 de mémorisation de la valeur du potentiel redox peuvent être répétées successivement selon une fréquence prédéfinie, par exemple toutes les 10 secondes, pour obtenir des valeurs mémorisées de potentiel redox, et le procédé peut comprendre en outre une étape 108 de calcul d'une valeur moyenne des valeurs mémorisées de potentiel redox. Cela permet d'établir une moyenne horaire ou journalière de la teneur en chlore libre dans la première solution 11.

Dans un autre mode de réalisation, la quatrième étape 104 de détermination de la valeur du potentiel redox et l'étape 107 de mémorisation de la valeur du potentiel redox sont répétées successivement jusqu'à ce que le pH de la seconde solution liquide 15 soit inférieur à une valeur de seuil prédéfinie, par exemple 12,4. Autrement dit, la mesure du potentiel redox et sa mémorisation sont stoppées dès que le pH de la seconde solution 15 est inférieur au seuil prédéfini. En effet, en dessous de la valeur de pH indiquée (12,4 dans l'exemple cité), la sonde redox n'est pas utilisable.

Avantageusement, le procédé selon l'invention comprend, une fois préalablement à la première étape 101, une étape 110 d'étalonnage d'une fonction reliant une concentration en chlore libre prédéterminée à une valeur du potentiel redox. Cette étape d'étalonnage permet de définir la fonction reliant la valeur du potentiel redox à la concentration en chlore libre dans la solution. Cette fonction est une relation mathématique. Lors de l'étape d'étalonnage, plusieurs points (par exemple 10 ou plus) de mesure sont effectués, par exemple en laboratoire, pour des solutions à différentes concentrations en chlore libre, par exemple de 300 à 1200 mg/L. Pour chacune de ces solutions dont on a mesuré traditionnellement la concentration en chlore libre, le potentiel redox de la solution à un pH adapté (par exemple à pH = 12,8) est également mesuré. En d'autres termes, pour chaque échantillon parmi différentes solutions, on mesure le potentiel redox mesuré à pH = 12,8 et la concentration en chlore libre. En reportant ces points sur un graphique représentant la concentration en chlore libre sur l'axe des ordonnées et le potentiel redox sur l'axe des abscisses, on obtient une courbe. Pour terminer l'étalonnage, il faut alors définir une fonction polynomiale présentant un bon coefficient de corrélation. Une fonction polynomiale d'ordre 2 de la forme y = a*x² + b*x + c (où y représente la concentration en chlore libre de la première solution liquide et x représente le potentiel redox de la seconde solution liquide, et a, b et c sont des coefficients) présente un très bon coefficient de corrélation. Dans une gamme de concentration en chlore libre entre approximativement 450 et 1000 mg/L, la relation mathématique suivante a été déterminée et a montré de très bons résultats, avec des valeurs pour a comprises entre 0 et 1, préférentiellement entre 0 et 0,5, des valeurs pour b comprises entre -500 et -100, préférentiellement entre -500 et -300, des valeurs pour c comprises entre 10000 et 60000, préférentiellement entre 50000et 60000. Toutefois, selon la sonde redox, la matrice d'eau utilisée et les conditions opératoires générales, les valeurs de a, b, c peuvent varier et éventuellement sortir des plages précédemment citées, sans sortir du cadre de l'invention.

Une fois cette relation mathématique établie, il est possible, en connaissant le potentiel redox (la valeur « x ») d'une solution à pH adapté de déterminer la concentration en chlore libre de la solution initiale (la valeur « y »).

L'étape d'étalonnage avec sa pluralité de mesures de concentration en chlore libre et de potentiels redox pour plusieurs échantillons se réalise une fois pour définir la relation mathématique. Par la suite, seule la relation mathématique est utilisée pour déterminer la concentration en chlore libre d'une solution à partir du potentiel redox de cette solution diluée avec de la soude.

L'étape d'étalonnage peut être réitérée ponctuellement, par exemple 1 à 2 fois par an, pour s'assurer du bon fonctionnement du procédé.

Le procédé de détermination de la concentration en chlore libre selon l'invention peut comprendre en outre une étape 109 de déclenchement d'une alarme 20 si au moins une des conditions suivantes est réalisée :
- La mesure du pH de la première solution liquide est inférieure à 7 ou supérieure à 13,
- La mesure du pH de la seconde solution liquide est inférieure à 12 ou supérieure à 13,
- La valeur du potentiel redox de la seconde solution liquide est inférieure à 0,1 V ou supérieure à 0,5 V.

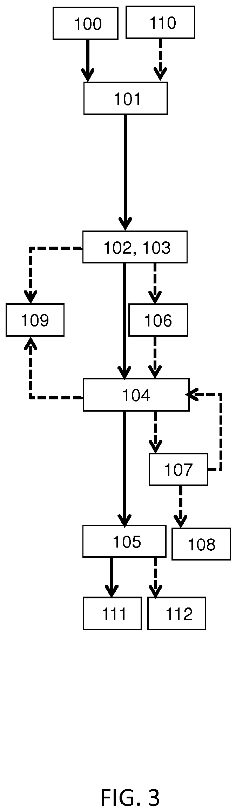

La **figure 3** représente schématiquement les étapes du procédé de régulation de la concentration en chlore libre d'un volume d'une première solution liquide dans une tour utilisant du chlore comme base active selon l'invention. Le procédé de régulation de la concentration en chlore libre du volume de la première solution liquide 11 dans une tour utilisant du chlore comme base active, comprend une étape 100 de prélèvement d'un échantillon du volume de la première solution liquide 11 en pied de la tour, puis les étapes du procédé de détermination de la concentration en chlore libre dans l'échantillon selon le procédé précédemment décrit. Et après la cinquième étape 105 de détermination de la concentration en chlore libre dans l'échantillon, le procédé de régulation comprend une étape 111 d'ajout d'une quantité d'un produit chloré dans le volume de la première solution liquide 11 en fonction de la concentration en chlore libre dans l'échantillon, de sorte à maintenir la concentration en chlore libre dans le volume de la première solution liquide 11 à une valeur prédéterminée.

Autrement dit, un premier échantillon est prélevé dans le réservoir en pied de tour. Il s'agit d'un échantillon de la première solution 11. On réalise sur cet échantillon le procédé de détermination de la concentration en chlore libre décrit précédemment. On obtient alors la concentration en chlore libre dans l'échantillon (c'est-à-dire correspondant à la première solution en pied de tour). Selon la valeur obtenue, du produit chloré est ajouté dans le réservoir en pied de tour pour maintenir la concentration en chlore libre dans le réservoir en pied de tour à une valeur prédéterminée, généralement à une valeur constante. Ainsi il est possible d'introduire de la solution de traitement (i.e. du produit chloré) pour maintenir constante la quantité d'oxydant dans la tour de désodorisation.

Par la suite, le procédé de régulation selon l'invention peut comprendre, après la cinquième étape 105 de détermination de la concentration en chlore libre dans la première solution liquide 11, une étape 112 de ré-injection de la seconde solution liquide 15 dans le volume de la première solution liquide 11, en pied de tour. L'effet de la soude ajoutée dans l'analyseur est négligeable du fait du petit échantillon prélevé par rapport au volume de première solution dans le réservoir en pied de tour. Alternativement, l'échantillon de la seconde solution 15 peut être dirigé vers l'exutoire, ce qui peut représenter une solution plus aisée à mettre en œuvre.

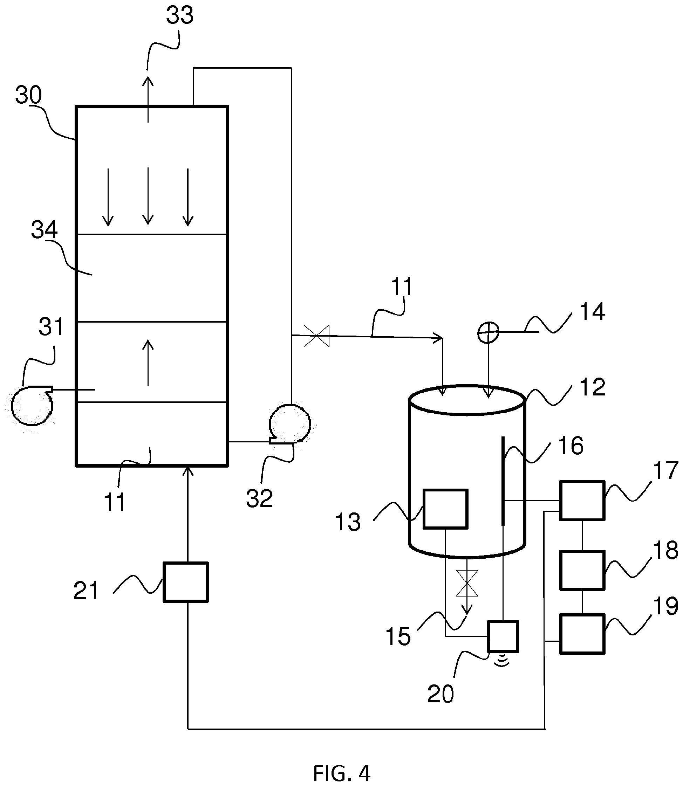

La **figure 4** représente schématiquement une tour de désodorisation 30 mettant en œuvre un dispositif 10 de détermination de la concentration en chlore libre selon l'invention. De l'air vicié extrait d'un ouvrage est envoyé grâce à un ventilateur 31 dans la tour 30, vers le haut de la tour. La première solution 11 se trouve dans le réservoir en pied de tour. Une pompe 32 puise la première solution 11 dans le réservoir en pied de tour et l'envoie vers le haut de la tour. La tour comprend un garnissage 34 permettant d'augmenter la surface de contact entre l'air vicié et la première solution qui ruisselle sur le garnissage 34. Une réaction chimique se produit entre l'air vicié et la première solution. Du chlore est consommé. Comme expliqué en introduction, pour assurer le bon fonctionnement de la tour de désodorisation, il est nécessaire d'ajuster la concentration en chlore libre dans la première solution 11. Afin de maintenir cette concentration à une valeur souhaitée (en général cette valeur est prédéfinie et constante), il faut ajouter du produit chloré (non représenté sur la figure). La quantité de produit chloré à ajouter se fait en fonction de la concentration en chlore libre dans le réservoir en pied de tour. Un échantillon de la première solution est prélevé. Sur la figure 4, ce prélèvement se fait dans la boucle de recirculation de la première solution vers le haut de la tour. Néanmoins, ce prélèvement peut aussi bien se faire directement au niveau du réservoir de la tour. Cet échantillon de première solution est ensuite dirigé vers le dispositif 10 de détermination de la concentration en chlore libre dans la première solution. Grâce au procédé de détermination de la concentration en chlore libre dans la première solution selon l'invention, la concentration en chlore libre dans cet échantillon est déterminée. Il est alors possible d'ajuster la concentration en chlore libre dans le réservoir en pied de tour.

L'invention permet de contrôler aisément et avec un investissement modéré la teneur en chlore libre dans une tour de désodorisation pour garantir le respect des normes sur la concentration en H2S en sortie du traitement de l'air tout en optimisant les consommations de réactifs de la tour concernée et de la tour en aval dont le rôle est d'éliminer les éventuels excès de chlore dans l'air rejeté.

## Revendications

1. Procédé de détermination de la concentration en chlore libre dans une première solution liquide (11), **caractérisé en ce qu'**il comprend les étapes suivantes :
• première étape (101) d'introduction de la première solution liquide (11) dans un analyseur (12) comprenant une sonde redox (16),
• deuxième étape (102) de mesure du pH de la première solution liquide (11),
• troisième étape (103) d'ajout de soude jusqu'à obtention d'une seconde solution liquide (15) de pH supérieur à 12, préférentiellement de 12,5 à 12,8, la troisième étape (103) étant préférentiellement mise en œuvre simultanément à la deuxième étape (102),
• quatrième étape (104) de détermination d'une valeur du potentiel redox de la seconde solution liquide (15) par la sonde redox (16),
• cinquième étape (105) de détermination de la concentration en chlore libre dans la première solution liquide (11) en fonction de la valeur du potentiel redox déterminé.

2. Procédé de détermination de la concentration en chlore libre selon la revendication 1, **caractérisé en ce qu'**il comprend entre la troisième étape (103) et la quatrième étape (104) une étape (106) de temporisation.

3. Procédé de détermination de la concentration en chlore libre selon l'une quelconque des revendications 1 ou 2, **caractérisé en ce qu'**il comprend après la quatrième étape (104) une étape (107) de mémorisation de la valeur du potentiel redox.

4. Procédé de détermination de la concentration en chlore libre selon la revendication 3, **caractérisé en ce que** la quatrième étape (104) de détermination de la valeur du potentiel redox et l'étape (107) de mémorisation de la valeur du potentiel redox sont répétées successivement selon une fréquence prédéfinie pour obtenir des valeurs mémorisées de potentiel redox, et **en ce que** le procédé comprend en outre une étape (108) de calcul d'une valeur moyenne des valeurs mémorisées de potentiel redox.

5. Procédé de détermination de la concentration en chlore libre selon la revendication 4, **caractérisé en ce que** la quatrième étape (104) de détermination de la valeur du potentiel redox et l'étape (107) de mémorisation de la valeur du potentiel redox sont répétées successivement jusqu'à ce que le pH de la seconde solution liquide (15) soit inférieur à une valeur de seuil prédéfinie.

6. Procédé de détermination de la concentration en chlore libre selon l'une quelconque des revendications précédentes, **caractérisé en ce qu'**il comprend, une fois préalablement à la première étape (101), une étape (110) d'étalonnage d'une fonction reliant une concentration en chlore libre prédéterminée à une valeur du potentiel redox.

7. Procédé de détermination de la concentration en chlore libre selon l'une quelconque des revendications précédentes, **caractérisé en ce qu'**il comprend en outre une étape (109) de déclenchement d'une alarme (20) si au moins une des conditions suivantes est réalisée :
- La mesure du pH de la première solution liquide est inférieure à 7 ou supérieure à 13,
- La mesure du pH de la seconde solution liquide est inférieure à 12 ou supérieure à 13,
- La valeur du potentiel redox de la seconde solution liquide est inférieure à 0,1 V ou supérieure à 0,5 V.

8. Procédé de régulation de la concentration en chlore libre d'un volume d'une première solution liquide (11) dans une tour utilisant du chlore comme base active, comprenant :
- une étape (100) de prélèvement d'un échantillon du volume de la première solution liquide (11) en pied de la tour,
- les étapes du procédé de détermination de la concentration en chlore libre dans l'échantillon selon l'une quelconque des revendications précédentes,
- après la cinquième étape (105) de détermination de la concentration en chlore libre dans l'échantillon, une étape (111) d'ajout d'une quantité d'un produit chloré dans le volume de la première solution liquide (11) en fonction de la concentration en chlore libre dans l'échantillon, de sorte à maintenir la concentration en chlore libre dans le volume de la première solution liquide (11) à une valeur prédéterminée.

9. Dispositif (10) de détermination de la concentration en chlore libre d'une première solution liquide (11), **caractérisé en ce qu'**il comprend :
• un analyseur (12), destiné à recevoir la première solution liquide (11),
• un pH-mètre (13) destiné à mesurer le pH de la première solution liquide (11) dans l'analyseur (12),
• un injecteur de soude (14) adapté à ajouter de la soude à la première solution liquide (11) jusqu'à obtention d'une seconde solution (15) de pH supérieur à 12, préférentiellement entre 12,5 et 12,8,
• une sonde redox (16), destinée à déterminer une valeur du potentiel redox de la seconde solution liquide (15),
• un calculateur (17) destiné à déterminer la concentration en chlore libre dans la première solution liquide (11) en fonction de la valeur du potentiel redox déterminé.

10. Dispositif (10) selon la revendication 9, **caractérisé en ce qu'**il comprend un moyen de mémorisation (18) de la valeur du potentiel redox.

11. Dispositif (10) selon l'une quelconque des revendications 9 ou 10, configuré pour déterminer la valeur du potentiel redox de la seconde solution liquide (15) et mémoriser la valeur du potentiel redox successivement et de façon répétée à une fréquence prédéfinie pour obtenir des valeurs mémorisées de potentiel redox, **caractérisé en ce qu'**il comprend un moyen de calcul (19) d'une valeur moyenne des valeurs mémorisées de potentiel redox.

12. Dispositif (10) selon l'une quelconque des revendications 9 à 11, **caractérisé en ce qu'**il comprend une alarme (20) configurée pour se déclencher si au moins une des conditions suivantes est réalisée :
- La mesure du pH de la première solution liquide est inférieure à 7 ou supérieure à 13,
- La mesure du pH de la seconde solution liquide est inférieure à 12 ou supérieure à 13,
- La valeur du potentiel redox de la seconde solution liquide est inférieure à 0,1 V ou supérieure à 0,5 V.

## Patentansprüche

1. Verfahren zur Bestimmung der Konzentration von freiem Chlor in einer ersten flüssigen Lösung (11), **dadurch gekennzeichnet, dass** es folgende Schritte umfasst:
• einen ersten Schritt (101) das Einleitens der ersten flüssigen Lösung (11) in einen Analysator (12), welcher eine Redox-Sonde (16) umfasst,
• einen zweiten Schritt (102) des Messens des pH-Wertes der ersten flüssigen Lösung (11),
• einen dritten Schritt (103) des Hinzufügens von Natriumhydroxid bis zum Erzielen einer zweiten flüssigen Lösung (15) mit einem pH-Wert über 12, vorzugsweise von 12,5 bis 12,8, wobei der dritte Schritt (103) vorzugsweise gleichzeitig mit dem zweiten Schritt (102) umgesetzt wird,
• einen vierten Schritt (104) des Bestimmens eines Wertes des Redox-Potenzials der zweiten flüssigen Lösung (15) durch die Redox-Sonde (16),
• einen fünften Schritt (105) das Bestimmens der Konzentration an freiem Chlor in der ersten flüssigen Lösung (11) in Abhängigkeit von dem Wert des bestimmten Redox-Potenzials.

2. Verfahren zur Bestimmung der Konzentration an freiem Chlor nach Anspruch 1, **dadurch gekennzeichnet, dass** es zwischen dem dritten Schritt (103) und dem vierten Schritt (104) einen Schritt (106) der Verzögerung umfasst.

3. Verfahren zur Bestimmung der Konzentration an freiem Chlor nach einem der Ansprüche 1 oder 2, **dadurch gekennzeichnet, dass** es nach dem vierten Schritt (104) einen Schritt (107) der Speicherung des Wertes des Redox-Potenzials umfasst.

4. Verfahren zur Bestimmung der Konzentration an freiem Chlor nach Anspruch 3, **dadurch gekennzeichnet, dass** der vierte Schritt (104) des Bestimmens des Wertes des Redox-Potenzials und der Schritt (107) des Speicherns des Wertes des Redox-Potenzials nacheinander entsprechend einer vorbestimmten Frequenz wiederholt werden, um gespeicherte Redox-Potenzialwerte zu erzielen, und dadurch, dass das Verfahren ferner einen Schritt (108) des Berechnens eines Mittelwertes der gespeicherten Redox-Potenzialwerte umfasst.

5. Verfahren zur Bestimmung der Konzentration an freiem Chlor nach Anspruch 4, **dadurch gekennzeichnet, dass** der vierte Schritt (104) des Bestimmens des Wertes des Redox-Potenzials und der Schritt (107) des Speicherns des Wertes des Redox-Potenzials nacheinander wiederholt werden, bis der pH-Wert der zweiten flüssigen Lösung (15) einen vorbestimmten Schwellenwert unterschreitet.

6. Verfahren zur Bestimmung der Konzentration an freiem Chlor nach einem der vorhergehenden Ansprüche, **dadurch gekennzeichnet, dass** es einmal vor dem Schritt (101), einen Schritt (110) des Eichens einer Funktion umfasst, welche eine vorbestimmte Konzentration an freiem Chlor mit einem Reduktion-Potenzialwert verbindet.

7. Verfahren zur Bestimmung der Konzentration an freiem Chlor nach einem der vorhergehenden Ansprüche, **dadurch gekennzeichnet, dass** es ferner einen Schritt (109) des Auslösens eines Alarms (20) umfasst, wenn mindestens eine der folgenden Bedingungen erfüllt ist:
- die Messung des pH-Wertes der ersten flüssigen Lösung weniger als 7 oder mehr als 13 beträgt,
- die Messung des pH-Wertes der zweiten flüssigen Lösung weniger als 12 oder mehr als 13,
- der Wert des Redox-Potenzials der zweiten flüssigen Lösung weniger als 0,1 V oder mehr als 0,5 V beträgt.

8. Verfahren zum Regulieren der Konzentration an freiem Chlor eines Volumens einer ersten flüssigen Lösung (11) in einem Turm, welcher Chlor als aktive Base verwendet, Folgendes umfassend:
- einen Schritt (100) des Entnehmens einer Probe aus dem Volumen der ersten flüssigen Lösung (11) am Fuße des Turms,
- die Schritte des Verfahrens zur Bestimmung der Konzentration an freiem Chlor in der Probe nach einem der vorhergehenden Ansprüche,
- nach dem fünften Schritt (105) des Bestimmens der Konzentration an freiem Chlor in der Probe, einen Schritt (111) des Hinzufügens einer Menge eines gechlorten Produkts in das Volumen der ersten flüssigen Lösung (11) in Abhängigkeit von der Konzentration an freiem Chlor in der Probe, um die Konzentration an freiem Chlor in dem Volumen der ersten flüssigen Lösung (11) auf einem vorbestimmten Wert zu halten.

9. Vorrichtung (10) zur Bestimmung der Konzentration von freiem Chlor einer ersten flüssigen Lösung (11), **dadurch gekennzeichnet, dass** sie Folgendes umfasst:
• einen Analysator (12), welcher zum Aufnehmen der ersten flüssigen Lösung (11) bestimmt ist,
• einen pH-Messer (13), welche zum Messen des pH-Wertes der ersten flüssigen Lösung (11) in dem Analysator (12) bestimmt ist,
• einen Natriumhydroxid-Einspritzer (14), welcher geeignet ist, Natriumhydroxid zu der ersten flüssigen Lösung (11) bis zum Erzielen einer zweiten Lösung (15) hinzuzufügen, deren pH-Wert mehr als 12 beträgt, vorzugsweise zwischen 12,5 und 12,8,
• eine Redox-Sonde (16), welche zum Bestimmen eines Wertes des Redox-Potenzials der zweiten flüssigen Lösung (15) bestimmt ist,
• einen Rechner (17), welcher zum Bestimmen der Konzentration an freiem Chlor in der ersten flüssigen Lösung (11) in Abhängigkeit von dem Wert des bestimmten Redox-Potenzials bestimmt ist.

10. Vorrichtung (10) nach Anspruch 9, **dadurch gekennzeichnet, dass** sie ein Mittel zum Speichern (18) des Redox-Potenzialwertes umfasst.

11. Vorrichtung (10) nach einem der Ansprüche 9 oder 10, welche konfiguriert ist, um den Wert des Redox-Potenzials der zweiten flüssigen Lösung (15) zu bestimmen und den Wert des Redox-Potenzials nacheinander und wiederholt mit einer vorbestimmten Frequenz zu speichern, um gespeicherte Redox-Potenzialwerte zu erzielen, dadurch charakterisiert, dass sie ein Mittel zum Berechnen (19) eines Mittelwertes der gespeicherten Redox-Potenzialwerte umfasst.

12. Vorrichtung (10) nach einem der vorhergehenden Ansprüche 9 bis 11, **dadurch gekennzeichnet, dass** sie einen Alarm (20) umfasst, welcher konfiguriert ist, um ausgelöst zu werden, wenn mindestens eine der folgenden Bedingungen erfüllt ist:
- die Messung des pH-Wertes der ersten flüssigen Lösung weniger als 7 oder mehr als 13 beträgt,
- die Messung des pH-Wertes der zweiten flüssigen Lösung weniger als 12 oder mehr als 13 beträgt,
- der Wert des Redox-Potenzials der zweiten flüssigen Lösung weniger als 0,1 V oder mehr als 0,5 V beträgt.

## Claims

1. A method for determining the free chlorine concentration in a first liquid solution (11), **characterized in that** it comprises the following steps:
• a first step (101) of introducing the first liquid solution (11) into an analyzer (12) comprising a redox probe (16),
• a second step (102) of measuring the pH of the first liquid solution (11),
• a third step (103) of adding sodium hydroxide until a second liquid solution (15) with a pH higher than 12, preferably from 12.5 to 12.8, is produced, the third step (103) being preferably carried out at the same time as the second step (102),
• a fourth step (104) of determining a value of the redox potential of the second liquid solution (15) by the redox probe (16),
• a fifth step (105) of determining the free chlorine concentration in the first liquid solution (11) according to the value of the determined redox potential.

2. The method for determining the free chlorine concentration according to claim 1, **characterized in that** it comprises a pause step (106) between the third step (103) and the fourth step (104).

3. The method for determining the free chlorine concentration according to any one of claims 1 or 2, **characterized in that** it comprises, after the fourth step (104) a step (107) of storing the value of the redox potential.

4. The method for determining the free chlorine concentration according to claim 3, **characterized in that** the fourth step (104) of determining the value of the redox potential and the step (107) of storing the value of the redox potential are repeated successively according to a predefined frequency so as to obtain stored redox potential values, and **in that** the method also comprises a step (108) of calculating a mean value of the stored redox potential values.

5. The method for determining the free chlorine concentration according to claim 4, **characterized in that** the fourth step (104) of determining the value of the redox potential and the step (107) of storing the value of the redox potential are repeated successively until the pH of the second liquid solution (15) is lower than a predefined threshold value.

6. The method for determining the free chlorine concentration according to any one of the preceding claims, **characterized in that** it comprises, once prior to the first step (101), a step (110) of calibrating a function linking a predetermined free chlorine concentration to a value of the redox potential.

7. The method for determining the free chlorine concentration according to any one of the preceding claims, **characterized in that** it also comprises a step (109) of triggering an alarm (20) if at least one of the following conditions is met:
- the measurement of the pH of the first liquid solution is lower than 7 or higher than 13,
- the measurement of the pH of the second liquid solution is lower than 12 or higher than 13,
- the value of the redox potential of the second liquid solution is lower than 0.1 V or higher than 0.5 V.

8. A method for regulating the free chlorine concentration of a volume of a first liquid solution (11) in a tower using chlorine as an active base, comprising:
- a step (100) of taking a sample of the volume of the first liquid solution (11) at the tower bottom,
- the steps of the method for determining the free chlorine concentration in the sample according to any one of the preceding claims,
- after the fifth step (105) of determining the free chlorine concentration in the sample, a step (111) of adding an amount of a chlorinated product to the volume of the first liquid solution (11) according to the free chlorine concentration in the sample, so as to maintain the free chlorine concentration in the volume of the first liquid solution (11) at a predetermined value.

9. A device (10) for determining the free chlorine concentration of a first liquid solution (11), **characterized in that** it comprises:
• an analyzer (12), intended to receive the first liquid solution (11),
• a pH-meter (13), intended to measure the pH of the first liquid solution (11) in the analyzer (12),
• a sodium hydroxide injector (14), intended to add sodium hydroxide to the first liquid solution (11) until a second solution (15) with a pH higher than 12, preferably between 12.5 and 12.8, is produced,
• a redox probe (16), intended to determine a value of the redox potential of the second liquid solution (15),
• a computer (17), intended to determine the free chlorine concentration in the first liquid solution (11) according to the value of the determined redox potential.

10. The device (10) according to claim 9, **characterized in that** it comprises a means for storing (18) the value of the redox potential.

11. The device (10) according to any one of claims 9 or 10, configured for determining the value of the redox potential of the second liquid solution (15) and storing the value of the redox potential successively and repeatedly at a predefined frequency in order to obtain stored redox potential values, **characterized in that** it comprises a means for calculating (19) a mean value of the stored redox potential values.

12. The device (10) according to any one of claims 9 to 11, **characterized in that** it comprises an alarm (20) configured to go off if at least one of the following conditions is met:
- the measurement of the pH of the first liquid solution is lower than 7 or higher than 13,
- the measurement of the pH of the second liquid solution is lower than 12 or higher than 13,
- the value of the redox potential of the second liquid solution is lower than 0.1 V or higher than 0.5 V.
